# EUROPÄISCHE PATENTSCHRIFT

(11) **EP 0 566 558 B1**
(45) Veröffentlichungstag und Bekanntmachung des Hinweises auf die Patenterteilung: **28.06.1995**
(21) Anmeldenummer: 93890067.7
(22) Anmeldetag: 01.04.1993
(51) Int. Cl.: F16F 15/16, F16C 3/02

(54) **Hohlwelle, insbesondere für Schiffsantriebe**
Hollow shaft, in particular for ship propulsion systems
Arbre creux, en particulier pour systèmes de propulsion de navire

(30) Priorität: 16.04.1992 AT 787/92
(43) Veröffentlichungstag der Anmeldung: 20.10.1993
(73) Patentinhaber: DR. ING. GEISLINGER & CO. SCHWINGUNGSTECHNIK GESELLSCHAFT M.B.H., A-5023 Salzburg (AT)
(72) Erfinder: Peifer, Peter, Dipl.-Ing., A-5020 Salzburg (AT)
(74) Vertreter: Hübscher, Heiner, Dipl.-Ing.

(56) Entgegenhaltungen:
- EP-A- 0 351 723
- DE-A- 3 024 636
- DE-U- 9 114 479
- FR-A- 1 123 737
- FR-A- 1 230 965
- FR-A- 2 600 013
- FR-A- 2 660 713
- US-A- 3 552 804
- PATENT ABSTRACTS OF JAPAN vol. 8, no. 258 (M-340)(1695) 27. November 1984 & JP-A-59 131 023

## Beschreibung

Die Erfindung bezieht sich auf eine Hohlwelle, insbesondere für Schiffsantriebe, bestehend aus einem Wellenmantel mit wenigstens einer schraubenlinienförmig ausgerichtete Elastizitätseigenschaften besitzenden Schale. Eine derartige Hohlwelle ist aus der EP-A-0 351 723 bekannt.

Antriebe, bei denen periodische Drehschwingungen auftreten, also beispielsweise alle Antriebe mit Kolbenmotoren, müssen mit besonderen Ausgleichs- und Dämpfungseinrichtungen ausgestattet sein, um resonanzbedingte Überbelastungen zu vermeiden und einen einwandfreien betrieb und die geforderte Standzeit sicherzustellen. Vor allem bei Schiffsantrieben, die mit sehr leistungsstarken Zweitaktmotoren und langen Antriebswellen arbeiten, ergeben sich extreme Verhältnisse, die auch sehr aufwendige, verhältnismäßig großdimensionierte Schwingungsdämpfer verlangen. Diese Schwingungsdämpfung erlaubt es, die Resonanzspitzen unter die zulässigen Belastungsgrenzen der verwendeten Werkstoffe, vor allem der Wellenwerkstoffe, zu drücken, wobei zusätzlich durch eine Verringerung der Steifigkeit der Werkstoffe die Resonanzspitzen in Richtung geringerer Drehzahlen verschoben werden könnten. Um dies zu erreichen, ist es bereits bekannt, in den Wellenstrang von Schiffsantrieben eine Zwischenwelle aus faserverstärktem Kunststoff einzusetzen, was nicht nur den Wellenstrang weicher macht, sondern auch dessen beträchtliches Gewicht reduziert. Die aus faserverstärktem Kunststoff hergestellten Wellen sind fertigungsbedingt Hohlwellen und können ein oder mehrschalig aufgebaut sein. Die Verstärkungsfasern sind schraubenlinienförmig verlegt und verlaufen kreuzweise oder von Schale zu Schale mit wechselnder Windungsrichtung, um Torsionsspannungen gleichmäßig aufnehmen zu können. Die Fasern sind in einem Kunstharz od. dgl. eingebettet, wodurch sich zwar eine gewisse Dämpfungseigenschaft ergibt, doch bleibt diese Dämpfungseigenschaft sehr gering und läßt sich praktisch nicht beeinflussen. Als wirkungsvolles Dämpfungselement innerhalb eines Antriebsstranges ist demnach eine solche Hohlwelle aus faserverstärktem Kunststoff nicht brauchbar.

Der Erfindung liegt daher die Aufgabe zugrunde, diese Mängel zu beseitigen und eine Hohlwelle der eingangs geschilderten Art zu schaffen, die als vollwertiger Schwingungsdämpfer einsetzbar ist.

Die Erfindung löst diese Aufgabe dadurch, daß die Schale eine Hohlkammer umschließt, die eine Dämpfungsflüssigkeit aufnimmt und über zumindest eine Drosselstelle mit einem Vorratsraum für Dämpfungsflüssigkeit in Strömungsverbindung steht. Eine solche Schale des Wellenmantels mit ihrer schraubenlinienförmigen Anisotropie wird durch eine Torsionsbelastung in Abhängigkeit von der Belastungsrichtung radial aufgeweitet oder zusammengedrückt, je nachdem, ob die Belastung in oder entgegen dem Schraubungssinne auftritt. Umschließt daher diese Schale einen mit Dämpfungsflüssigkeit gefüllten Hohlraum, der an einen entsprechenden Vorratsraum angeschlossen ist, kommt es bei Torsionsbelastung wegen der damit verbundenen Volumsänderung zu einem Verdrängen oder Ansaugen der Flüssigkeit und im Falle einer Drehschwingung im Rhythmus der Drehschwingungsbelastung zu einer atmenden Pumpbewegung mit dem erzwungenen Durchfluß der Dämpfungsflüssigkeit durch die Drosselstelle. Diese Drosselung garantiert die gewünschte Schwingungsdämpfung. Der Dämpfungseffekt läßt sich dabei ohne Schwierïgkeiten durch die Zahl und/oder Querschnittsgröße der Drosselstellen, durch die Eigenschaften der Dämpfungsflüssigkeit, durch das Volumen der Hohlräume, durch den Verlauf, die Stärke und Richtung der Anisotropie in den Schalen u. dgl. innerhalb weiter Grenzen beeinflussen, so daß eine Hohlwelle mit ausgezeichneter, gut einstellbarer Dämpfungscharakteristik vorliegt. Diese Hohlwelle kann beliebig eingesetzt und in den verschiedensten Antriebssystemen verwendet werden und macht zumindest größere Drehschwingungsdämpfer auch bei Schiffsantrieben unnötig.

Die erforderliche Anisotropie der Schalen läßt sich auf verschiedene Weise entweder durch eine spezielle Wahl des Werkstoffes oder auch durch eine formgebende Gestaltung erreichen. Vorteilhafterweise können daher auch die Schalen aus faserverstärktem Kunststoff bestehen, dessen entlang von Schraubenlinien verlegte Verstärkungsfasern eine bevorzugte Schraubungsrichtung aufweisen, oder die Schalen können eine schraubenlinienförmige Oberflächenstruktur, beispielsweise schraubenlinienförmig verlaufende Nuten oder Rippen, aufweisen, um durch die damit gewonnene Anisotropie bei Drehschwingungsbelastung den angestrebten Atmungseffekt zu erzielen. Ob dazu eine stoffliche oder strukturelle Anisotropie gewählt wird, kann von den gewünschten zusätzlichen Eigenschaften des Materials, wie Steifigkeit oder Weichheit, Festigkeit, Gewicht, Beständigkeit u. dgl. abhängig gemacht werden.

Grundsätzlich ist es durchaus möglich, nur eine Schale vorzusehen und den Wellenhohlraum als dämpfungsflüssigkeitsgefüllte Hohlkammer auszubilden, wozu eine Leitungsverbindung zu einem außerhalb der Welle oder in einem axial anschließenden Wellenabschnitt angeordneten Vorratsraum erforderlich wäre. besonders günstig ist es jedoch, wenn der Wellenmantel wenigstens zwei unter Freilassung jeweils eines die Hohlkammer bildenden Zwischenraumes beiderends miteinander dicht verbundene Schalen umfaßt, wobei die Elastizitätseingenschaften jeweils benachbarter Schalen gegensinnig zueinander orientiert sind, da durch diese gegensinnige Anisotropie bei Torsionsbelastung ein gegengleiches Aufwelten oder Zusammendrücken der Schalen erfolgt und sich der Atmungs-und Pumpeffekt und damit die Dampfungswirkung wesentlich verstärkt. Außerdem lassen sich dabei die als Hohlkammern dienenden Zwischenräume zusammen mit dem Vorratsraum im gleichen Wellenabschnitt unterbringen.

Bildet der Hohlraum innerhalb des Wellenmantels den Vorratsraum und sind Radialbohrungen zwischen den Hohl- und Zwischenräumen vorgesehen, entsteht eine vor allem für einen zweischaligen Wellenmantel geeignete Konstruktion, da der vorhandene Zwischenraum direkt mit dem den Vorratsraum bildenden Hohlraum der Welle zusammenwirkt und die Dämpfungsflüssigkeit aufgrund der Fliehkraft problemlos in den Zwischenraum zurückströmen kann. Es ist kein außerhalb der Welle angeordneter Vorratsraum erforderlich, was selbstverständlich bei einer entsprechenden Leitungsverbindung zwischen Vorratsraum und Zwischenraum möglich wäre, und es kommt zu einer einfachen, in sich geschlossenen Wellenausführung.

Besteht der Wellenmantel zumindest aus drei Schalen und bilden die über Durchströmöffnungen miteinander verbundenen Zwischenräume wechselweise die Hohlkammern und Vorratsräume, kann auf einen eigenen Vorratsraum verzichtet werden, da ja die Zwischenräume durch ihre atmende gegensinnige Pumpbewegung die Dämpfungsflüssigkeit durch die Durchströmöffnungen wechselweise von einem Zwischenraum zum anderen pumpen. Es entsteht eine sehr kompakte Dämpfungseinrichtung mit ausgezeichneter Dämpfungseigenschaft.

Vorteilhaft ist es, wenn Drosselstellen mit verstellbarem Durchströmquerschnitt vorgesehen sind, so daß sich durch die Wahl des jeweiligen Durchströmquerschnittes der Dämpfungseffekt beeinflussen läßt. Zum Verstellen der Durchströmquerschnitte können auf einfache Weise in die vorhandenen Durchströmöffnungen verschieden dimensionierte Büchsen, Durchlaßschrauben unterschiedlicher Bohrungsgrößen, aber auch Strömungsorgane mit betätigbaren Schieber- oder Ventileinsätzen Verwendung finden.

In der Zeichnung ist der Erfindungsgegenstand rein schematisch veranschaulicht, und zwar zeigen
- Fig. 1 und 2: zwei Ausführungsbeispiele einer erfindungsgemäßen Hohlwelle jeweils im Axialschnitt,
- Fig. 3: die Hohlwelle nach Fig. 2 in teilgeschnittener Ansicht und
- Fig. 4: ein weiteres Ausführungsbeispiel einer erfindungsgemäßen Hohlwelle im Axialschnitt.

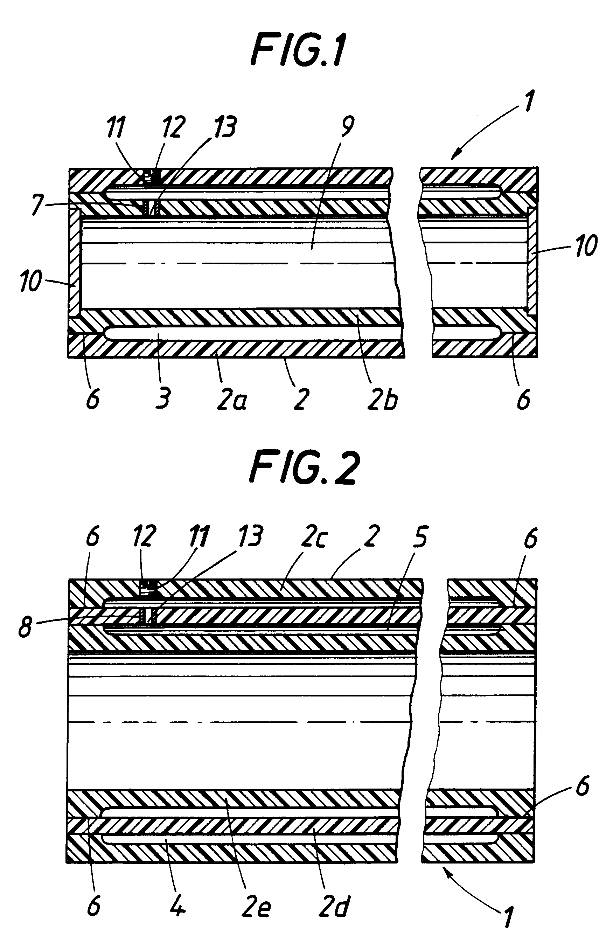

Eine Hohlwelle 1 weist einen Wellenmantel 2 aus wenigstens einer Schale 2a,b,c,d,e,f,g auf, die jeweils eine schraubenlinienförmige Anisotropie besitzt. Gemäß den Ausführungsbeispielen nach Fig. 1 - 3 sind zwei bzw. drei Schalen 2a,b; 2c,d,e unter Freilassung jeweils eines Zwischenraumes 3, 4, 5 beiderends miteinander dicht, beispielsweise durch Klebungen 6, verbunden. Die Schalen 2a - e bestehen aus faserverstärktem Kunststoff, wobei die Verstärkungsfasern im wesentlichen entlang von Schraubenlinien verlaufen und es für jede Schale eine bevorzugte Schraubungsrichtung, also entweder eine linksdrehende oder rechtsdrehende Schraubungsrichtung S , S gibt. Die bevorzugten Schraubungsrichtungen benachbarter Schalen sind allerdings gegensinnig zueinander orientiert, so daß die Schraubenlinien der hintereinandergereihten Schalen abwechselnd links- oder rechtsdrehend verlaufen.

Aufgrund dieses Faserverlaufes ergibt sich eine schraubenlinienförmige Anisotropie für jede Schale und jede Schale wird bei einer Drehbelastung im Sinne der Schraubungsrichtung gewissermaßen zusammengedreht und dadurch radlal zusammengedrückt, bei einer Drehbelastung entgegen der Schraubungsrichtung aber aufgedreht und radial aufgeweitet. Da benachbarte Schalen Fasern mit gegensinnig zueinander verlaufender Anisotropie aufweisen, werden die einzelnen Schalen bei Drehbelastung des Wellenmantels in Abhängigkeit ihrer Anisotrople aufgeweitet bzw. zusammengedrückt und die vorhandenen Zwischenräume 3, 4, 5 zwischen den einzelnen Schalen dadurch im Rhythmus der Schwingungsbelastung verengt bzw. aufgeweitet. Werden die als Hohlkammern ausgebildeten Zwischenräume 3, 4, 5 nun mit einer Dämpfungsflüssigkeit gefüllt und über Drosselstellen 7, 8 an einen Vorratsraum für Dämpfungsflüssigkeit angeschlossen, kommt es zu einer Pumpbewegung für die Dämpfungsflüssigkeit und die Flüssigkeit wird über die Drosselstellen 7, 8 vom jeweiligen Zwischenraum in den Vorratsraum gedrückt oder vom Vorratsraum in den Zwischenraum zurückgesaugt. Das Drosseln der Strömungsbewegung führt dabei zu einer wirkungsvollen Schwingungsdämpfung.

Beim Ausführungsbeispiel nach Fig. 1 umfaßt der Wellenmantel 2 zwei Schalen 2a, 2b, wobei der Zwischenraum 3 als Hohlkammer über eine die Drosselstelle bildende Radlalbohrung 7 mit dem Hohlraum 9 der Hohlwelle 1 in Strömungsverbindung steht, welcher Hohlraum 9 als Vorratsraum für die Dämpfungsflüssigkeit dient. Dazu braucht dieser Hohlraum lediglich an den Stirnseiten mit geeigneten Abdeckungen 10 verschlossen zu werden, und die Hohlwelle ist als Dämpfer einsatzbereit. Zum Einfüllen der Dämpfungsflüssigkeit gibt es in der Außenschale 2a, eine Füllöffnung 11, die koaxial zur Radialbohrung 7 verläuft, so daß die Herstellung dieser Bohrungen keine Schwierigkeiten bereitet. Außerdem läßt sich dann durch die über einen entsprechenden Verschluß 12 verschließbare Füllöffnung 11 in der Radialbohrung 7 wahlweise eine eigene Drossel 13 einsetzen, die dann eine Änderung des Durchströmquerschnittes der Drosselstelle erlaubt.

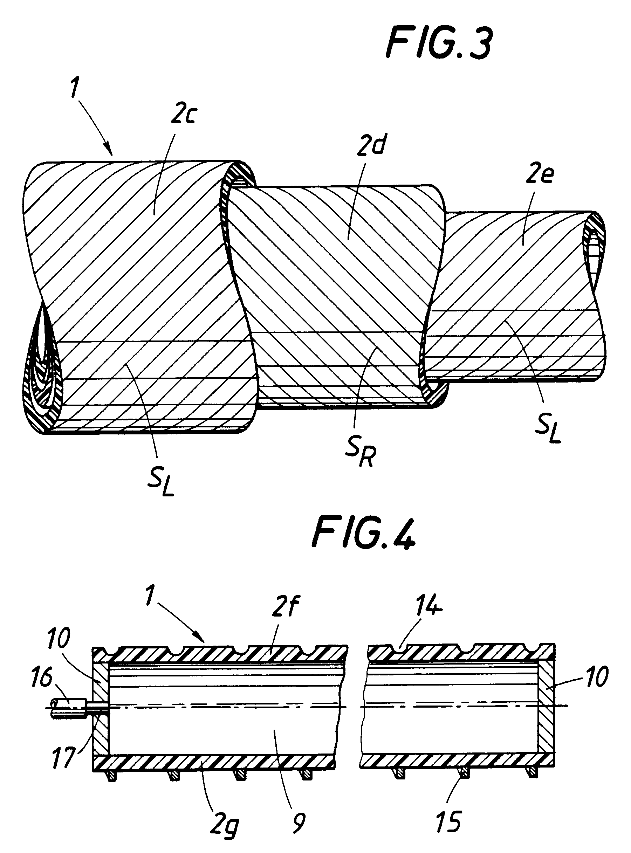

Beim Ausführungsbeispiel nach Fig. 2 und 3 sind drei Schalen 2c, 2d, 2e miteinander verklebt und die beiden Zwischenräume 4, 5, sind über Durchströmöffnungen 8 in der mittleren Schale 2d miteinander strömungsverbunden. Dadurch können die Zwischenräume sowohl als Hohlkammern als auch als Vorratsräume verwendet werden, da bei einer Schwingungsbelastung die Zwischenräume 4, 5 abwechselnd verengt bzw. aufgeweitet werden und daher die aus dem sich verengenden Zwischenraum verdrängte Dämpfungsflüssigkeit im sich aufweitenden anderen Zwischenraum aufgenommen wird. bei Schwingungsbelastung kommt es zu einem gegengleichen Atmen der Zwischenräume 4, 5 und die durch die Durchströmöffnung 8 als Drosselstelle hin- und herströmende Dämpfungsflüssigkeit bewirkt den gewünschten Dämpfungseffekt. Auch hier ist eine entsprechende Füllöffnung 11 vorgesehen, die das befüllen der Zwischenräume 4, 5 und das Herstellen der Durchströmöffnungen 8 ermöglicht, wobei in die Durchströmöffnung 8 wieder eine eigene Drossel 13 zur einfachen Beeinflussung der Dämpfungseigenschaften einsetzbar ist.

Gemäß dem Ausführungsbeispiel nach Fig. 4 wird die schraubenlinienförmige Anisotropie der Schalen nicht durch einen Werkstoff mit orthotropen Eigenschaften, dessen Orthotropiehauptachsen tangential zu Schraubenlinien verlaufen, erreicht, sondern durch eine besondere formgebende Gestaltung der Schalen. Die dargestellte Hohlwelle 1 weist beisplelsweise einen Wellenmantel aus nur einer Schale 2f, 2g auf, die schraubenlinienförmig sich erstrechende Nuten 14 (obere Hälfte) oder Rippen 15 (untere Hälfte) bildet und dadurch die gewünschte Anisotropie besitzt. Der Hohlraum 9 der Welle dient hier als mit Dämpfungsflüssigkeit gefüllte Hohlkammer, die über eine nur angedeutete Verbindungsleitung 16 mit einem außerhalb angeordneten, nicht dargestellten Vorratsraum in Strömungsverbindung steht. Eine Drehschwingungsbelastung führt wegen der Schalenanisotropie wiederum zu einer Atmungsbewegung, die Dämfungsflüssigkeit durch eine Drosselstelle 17, beispielsweise die Anschlußbohrung für die Verbindungsleitung in einer der den Wellenhohlraum 9 abschließenden Abdeckungen 10, zwischen Hohlraum 9 und Vorratsraum hin- und herpumpt und eine Schwingungsdämpfung verursacht.

## Patentansprüche

1. Hohlwelle (1), insbesondere für Schiffsantriebe, bestehend aus einem Wellenmantel (2) mit wenigstens einer schraubenlinienförmig ausgerichtete Elastizitätseigenschaften besitzenden Schale (2a - g), dadurch gekennzeichnet, daß die Schale (2a - g) eine Hohlkammer (9) umschließt, die eine Dämpfungsflüssigkeit aufnimmt und über zumindest eine Drosselstelle (7, 8, 15, 17) mit einem Vorratsraum für Dämpfungsflüssigkeit in Strömungsverbindung steht.

2. Hohlwelle nach Anspruch 1, dadurch gekennzeichnet, daß die Schalen (2a - e) aus faserverstärktem Kunststoff bestehen, dessen entlang von Schraubenlinien verlegte Verstärkungsfasern eine bevorzugte Schraubungsrichtung aufweisen.

3. Hohlwelle nach Anspruch 1, dadurch gekennzeichnet, daß die Schalen (2f, 2g) eine schraubenlinienförmige Oberflächenstruktur, beispielsweise schraubenlinienförmig verlaufende Nuten (14) oder Rippen (15) aufweisen.

4. Hohlwelle nach einem der Ansprüche 1 - 3, dadurch gekennzeichnet, daß der Wellenmantel (2) wenigstens zwei unter Freilassung jeweils eines die Hohlkammer bildenden Zwischenraumes (3, 4, 5) beiderends miteinander dicht verbundene Schalen (2a - e) umfaßt, wobei die Elastizitätseigenschaften jeweils benachbarter Schalen gegensinnig zueinander orientiert sind.

5. Hohlwelle nach Anspruch 4, dadurch gekennzeichnet, daß der Hohlraum (9) innerhalb des Wellenmantels (2) den Vorratsraum bildet und Radialbohrungen (7) zwischen den Hohl- und Zwischenräumen (9, 3) vorgesehen sind.

6. Hohlwelle nach Anspruch 4, dadurch gekennzeichnet, daß der Wellenmantel (2) zumindest aus drei Schalen (2c - e) besteht und die über Durchströmöffnungen (8) miteinander verbundenen Zwischenräume (4, 5) wechselweise die Hohlkammern und Vorratsräume bilden.

7. Hohlwelle nach einem der Ansprüche 1 - 6, dadurch gekennzeichnet, daß Drosselstellen (7, 8, 13, 17) mit verstellbarem Durchströmquerschnitt vorgesehen sind.

## Claims

1. A hollow shaft (1), more particularly for marine propulsion units, consisting of a shaft jacket (2) comprising at least one bush (2a - g) having helically aligned elasticity properties, characterised in that the bush (2a - g) encloses a hollow chamber (9) which receives a damping fluid and communicates in terms of flow with a damping fluid reservoir chamber via at least one restrictor (7, 8, 13, 17).

2. A hollow shaft according to claim 1, characterised in that the bushes (2a - e) consist of fibre-reinforced plastic, whose reinforcing fibres disposed along helixes have a preferred helical direction.

3. A hollow shaft according to claim 1, characterised in that the bushes (2f, 2g) have a helical surface structure, e.g. helically extending grooves (14) or ribs (15).

4. A hollow shaft according to any one of claims 1 to 3, characterised in that the shaft jacket (2) comprises at least two bushes (2a - e) sealingly interconnected at both ends, an intermediate space (3, 4, 5) which forms the hollow chamber being left free in each case, the elasticity properties of adjacent bushes being oriented in opposite directions to one another.

5. A hollow shaft according to claim 4, characterised in that the cavity (9) inside the shaft jacket (2) forms the reservoir chamber and radial bores (7) are provided between the cavity and the intermediate spaces (9, 3).

6. A hollow shaft according to claim 4, characterised in that the shaft jacket (2) consists of at least three bushes (2c - e) and the intermediate spaces (4, 5) interconnected via throughflow apertures (8) alternately form the hollow chambers and reservoir chambers.

7. A hollow shaft according to any one of claims 1 to 6, characterized in that restrictors (7, 8, 13, 17) having an adjustable throughflow cross-section are provided.

## Revendications

1. Arbre creux (1) pour propulsion de navires, composé d'une enveloppe d'arbre (2), avec au moins une coque (2a à g) ayant des propriétés d'élasticité orientées selon une hélicoïde,
caractérisé en ce que la coque (2a à g) enclôt une chambre creuse (9), recevant un liquide d'amortissement et reliée hydrauliquement à une enceinte de stockage pour le liquide d'amortissement par l'intermédiaire d'au moins un point d'étranglement (7, 8, 13, 17).

2. Arbre creux selon la revendication 1,
caractérisé en ce que les coques (2a à e) sont réalisées en matière synthétique renforcée par des fibres, dont les fibres de renforcement, posées en suivant des hélicoïdes, présentent une direction de vissage préférentielle.

3. Arbre creux selon la revendication 1,
caractérisé en ce que les coques (2f, 2g) présentent une structure de surface hélicoïde par exemple des gorges (14) ou nervures (15) s'étendant en suivant des hélices.

4. Arbre creux selon l'une des revendications 1 à 3,
caractérisé en ce que l'enveloppe d'arbre (2) comprend au moins deux coques (2a à e), reliées de façon étanche aux deux extrémités et laissant subsister chacune un espace intermédiaire (3, 4, 5) constituant la chambre creuse, les propriétés d'élasticité de chacune des coques voisines étant orientées à contre-sens les unes par rapport aux autres.

5. Arbre creux selon la revendication 4,
caractérisé en ce que l'espace creux (9) constitue à l'intérieur de l'enveloppe d'arbre (2) l'enceinte de stockage et des perçages radiaux (7) étant prévus entre les espaces creux et intermédiaires (9, 3).

6. Arbre creux selon la revendication 4,
caractérisé en ce que l'enveloppe d'arbre (2) est composée d'au moins deux coques (2c à e) et les espaces intermédiaires (4, 5) reliés ensemble par des ouvertures de passage d'écoulement (8) constituant alternativement les chambres creuses et les enceintes de stockage.

7. Arbre creux selon l'une des revendications 1 à 6,
caractérisé en ce que des points d'étranglement (7, 8, 13, 17) à section transversale de passage d'écoulement réglable sont prévus.
